# EUROPEAN PATENT APPLICATION

(11) **EP 3 301 173 A1**
(43) Date of publication of application: **04.04.2018**
(21) Application number: 16191542.6
(22) Date of filing: 29.09.2016
(51) Int. Cl.: C12N 1/04, C12N 1/20, A61K 35/747

(54) **GROWTH MEDIUM AND PROCESS FOR PRODUCING LACTIC ACID BACTERIA**

(71) Applicant: Nestec S.A., 1800 Vevey (CH)
(72) Inventor: ANANTA, Edwin, 268824 SINGAPORE (SG); BERTOLI, Constantin, 3510 KONOLFINGEN (CH); BOGICEVIC, Biljana, 3012 BERN (CH); BOURQUI, Bertrand, 3013 BERN (CH); BRAUN, Marcel, 3510 KONOLFINGEN (CH)
(74) Representative: Künzi, Sophie

(57) **Abstract**

The present invention relates to the production of lactic acid bacteria and to a growth medium comprising yeast extract, at least one carbohydrate and lecithin that can be used in such process. Lecithin is used as a replacement of polyoxyethylene-sorbitan-mono-oleate in bacterial growth media leading to high yield of lactic acid bacteria, while ensuring that the obtained bacteria can be consumed by the most sensitive consumers.

## Description

### Field of the invention

The present invention relates to the production of lactic acid bacteria and to a growth medium comprising yeast extract, at least one carbohydrate and lecithin that can be used in such process. Lecithin is used as a replacement of polyoxyethylene-sorbitan-mono-oleate in bacterial growth media leading to high yield of lactic acid bacteria, while ensuring that the obtained bacteria can be consumed by the most sensitive consumers.

### Background of the invention

Lactic acid bacteria are extensively used in the food and beverages industry, namely as probiotics. The production of lactic acid bacteria being an expensive process, there is continuous research to improve the growth media used to produce these bacteria with high yield.

As lactic acid bacteria are commonly used in products for sensitive consumers, like infants, small children, paediatric subjects, pregnant or lactating women, elderly people or patients in hospitals, it is in addition desirable that the growth medium be devoid of any compound that may be harmful for the individual consuming a product incorporating the lactic acid bacteria. Accordingly, regulatory authorities keep tight control of the ingredients that are allowed in the growth medium of lactic acid bacteria for use in products intended for sensitive consumers, such as for example infant formula.

One standard bacterial growth medium for lactic acid bacteria is the MRS medium. This growth medium is particularly well suited for the growth of lactic acid bacteria and namely for those of the genus *Lactobacillus.* This medium comprises as main components yeast extract, at least one carbohydrate, such as a sugar, and polyoxyethylene-sorbitan-mono-oleate, which combination enhances the growth of lactic acid bacteria such as *Lactobacilli.* Polyoxyethylene-sorbitan-mono-oleate (CAS Registery Number: 9005-65-6), also named polysorbate 80 and commercially available as Tween^{®} 80 from Sigma Aldrich, has been identified as potentially related with health problems. For example, polyoxyethylene-sorbitan-mono-oleate has been reported as having potential harmful effects on the central nerve system of rats (Brubaker et al; Effect of Tween 80 on exploratory behavior and locomotor activity in rats, Life Sciences, vol 30, p. 1965-1971), as having potential toxic effects on the liver, heart and kidney in rats (Nityanand et al., Effect of chronic oral administration of Tween 80 in Charles Foster Rats, Indian J. Med. Res, 69:664-670 (1979)). In human, it has even been associated with fatalities of low birth weight premature infants (Alade et al, Polysorbate 80 and E-Ferol Toxicity; Pediatrics; 77(4):593-597 (1986)).

It is therefore desirable to reduce the amount of polyoxyethylene-sorbitan-mono-oleate and even better to substantially or even totally avoid this substance in bacterial growth media such as the MRS medium, while maintaining or even improving the yield of the bacteria. Simple removal of polyoxyethylene-sorbitan-mono-oleate from the growth medium unfortunately very negatively impacts the growth of the bacteria, some strains being even not able to grow at all in MRS medium devoid of polyoxyethylene-sorbitan-mono-oleate. Also, removing polyoxyethylene-sorbitan-mono-oleate from the growth medium reduces the resistance of lactic acid bacteriae to a drying step, such as spray-drying.

The above-mentioned problems have been addressed in WO 2010/100047. In this document, polyoxyethylene-sorbitan-mono-oleate has been replaced by diacetyl tartaric acid esters of mono- and di-glycerides (DATEM). DATEM is however not sufficiently safe to be used in sensitive products such as infant formula, rendering the solution described in this document unsuitable for such applications and making it desirable to find safer replacement of polyoxyethylene-sorbitan-mono-oleate in bacterial growth media that could even be used in infant formula for example.

The present invention advantageously solves the above-mentioned problems.

### Summary of the invention

In a first aspect, the invention provides a bacterial growth medium comprising yeast extract, at least one carbohydrate and lecithin.

In a second aspect, the invention provides a process for producing a lactic acid bacteria biomass comprising
a. fermenting at least one strain of lactic acid bacteria in a bacterial growth medium of the invention; and
b. harvesting the bacteria.

In a third aspect, the invention provides a bacterial culture comprising lactic acid bacteria in a bacterial growth medium of the invention.

In a fourth aspect, the invention relates to the use of lecithin to stimulate the growth of lactic acid bacteria in a growth medium, wherein the growth medium comprises yeast extract and at least one carbohydrate and comprises less than 0.1wt% of polyoxyethylene-sorbitan-mono-oleate.

In a fifth aspect, the invention provides a lactic acid bacteria biomass obtainable or obtained by the process of the invention.

In a sixth aspect, the invention provides a product comprising a lactic acid bacteria biomass of the invention.

In a seventh aspect, the invention relates to the use of ecithin to improve the survival of lactic acid bacteria during drying, storage and/or reconstitution with a liquid, wherein lecithin is added to the growth medium of the lactic acid bacteria, wherein the growth medium comprises yeast extract and at least one carbohydrate and wherein the growth medium comprises less than 0.1wt% of polyoxyethylene-sorbitan-mono-oleate.

### Brief Description of the Drawings

**Figure 1****:** Graphic representation of the fermentation yield of *Lactobacillus johnsonii* deposited as CNCM I-1225with 0.3% of Tween^{®} 80, and with various concentrations of the lecithin Emulpur^{®} IP (0.1%, 0.2%, 0.3%, 0.4%, 0.5% and 0.6%), as a replacement of Tween^{®} 80.
**Figure 2****:** Graphic representation of the fermentation yield of *Lactobacillus bulgaricus* deposited as ATCC 11842 with 0.1 % of Tween^{®} 80, without Tween^{®} 80 and with various concentrations of the lecithin Emulpur^{®} IP (0.1%, 0.2%, 0.3%), as a replacement of Tween^{®} 80.
**Figure 3****:** Graphic representation of the fermentation yield of *Lactobacillus curvatus* deposited as ATCC 25601 with 0.1 % of Tween^{®} 80, without Tween^{®} 80 and with various concentrations of the lecithin Emulpur^{®} IP (0.1%, 0.2%, 0.3%), as a replacement of Tween^{®} 80.
**Figure 4****:** Graphic representation of the fermentation yield of *Lactobacillus acidophilus* deposited as ATCC4356 with 0.1 % of Tween^{®} 80, without Tween^{®} 80 and with various concentrations of the lecithin Emulpur^{®} IP (0.1%, 0.2%, 0.3%), as a replacement of Tween^{®} 80.

### Detailed description of the invention

### Bacterial growth medium

The bacterial growth medium of the invention is particularly advantageous in that it allows efficient growth of a wide variety of lactic acid bacteria, even in the absence of polyoxyethylene-sorbitan-mono-oleate (Tween^{®} 80), which is of potential risk for an individual consuming the obtained bacteria, such as an infant. In particular similar or, for some strains, even higher yields are obtained when a standard amount of Tween^{®} 80 is replaced by lecithin. The growth medium is also effective in making the cultured bacteria more resistant during subsequent processing, such as to improve the bacteria's survival during drying, during storage and/or upon reconstitution in a liquid, preferably in water.

The bacterial growth medium of the invention comprises yeast extract, at least one carbohydrate and lecithin. The yeast extract and the carbohydrate are the essential components of, for example, the standard MRS medium. In such media, polyoxyethylene-sorbitan-mono-oleate (Tween^{®} 80) is normally used. In the growth medium of the present invention, polyoxyethylene-sorbitan-mono-oleate is replaced, in whole or in part, by lecithin. Thus, in a preferred aspect of the invention, the medium has reduced content of polyoxyethylene-sorbitan-mono-oleate compared to a standard medium. Preferably, the growth medium comprises less than 0.1wt%, more preferably less than 0.05wt%, more preferably less than 0.01wt% of polyoxyethylene-sorbitan-mono-oleate. In a most preferred embodiment, the growth medium is completely devoid of polyoxyethylene-sorbitan-mono-oleate.

Yeast extract and the carbohydrate, in combination with lecithin, are the key nutrients needed by the lactic acid bacteria to grow.

Lecithin is particularly advantageous because it is safe for use in products for most sensitive consumers, such as infants, including preterm infants or paediatric subjects. Thus no risk is associated to the consumption of lactic acid bacteria such as probiotics grown in a growth medium with lecithin.

Lecithin has its common meaning in the art. It refers to complex mixtures of lipids, namely phospho- and glycolipids. It also contains other minor components, such as carbohydrates. Any lecithin can be used for the purpose of the present invention. One preferred lecithin type is de-oiled lecithin. De-oiled lecithin advantageously has an improved dispersibility in the growth medium, which increases the availability of the lecithin for the lactic acid bacteria. For the same reason, lecithin in powder form, more preferably de-oiled lecithin in powder form, is particularly advantageous.

Lecithin suitable for use in the growth medium of the present invention may originate from diverse sources such as egg yolk or vegetable sources, preferably vegetable sources, such as for example soya, sunflower or low erucic acid rapeseed (also known as canola). Lecithin having high oleic acid content, low linoleic acid content and/or low a-linolenic acid content, from any of these sources, can advantageously be used. Fractionated lecithin, as well as lysolecithin, can also advantageously be used for the purpose of the present invention.

Concentrations of lecithin as small as 0.1wt%, based on the total weight of the growth medium, have proven efficient to obtain industrially usable yields of lactic acid bacteria in the medium of the invention. Concentrations higher than 0.1wt% work as well, but the yield does not increase as a function of the concentration of lecithin. The optimal concentration of lecithin to be used may vary from strain to strain and can be determined by trial and error, based on the present description. The practical maximum concentration of lecithin that can be used is defined as the maximum amount that can be dispersed in the growth medium. Such highest concentration may vary depending on the exact composition of the medium, but the person skilled in the art will easily recognize when too high an amount of lecithin is used because the lecithin cannot be suitably dispersed. Preferably, the maximum concentration that can be used is 5wt%, preferably 3wt%, more preferably 2wt%, even more preferably 1wt%, most preferably 0.6wt%. Thus lecithin should be present in an amount of at least 0.1wt%, preferably in an amount of 0.1wt% to 5wt%, more preferably of 0.1wt% to 3wt%, more preferably of 0.1 to 2wt%, even more preferably of 0.1 to 1wt%, most preferably in an amount of 0.1 to 0.6wt%, based on the total weight of the growth medium.

Besides lecithin, the other components of the medium are those commonly used in the art in growth media for lactic acid bacteria, such as the MRS medium or media derived from the MRS medium.

Suitable yeast extracts are well known to the person skilled in the art. Lactic acid bacteria need a source of nitrogen to grow. Yeast extract is one preferred source of nitrogen for lactic acid bacteria, particularly suited to grow lactic acid bacteria with high yield. Any type of yeast extract commonly used in growth media for lactic acid bacteria can be used. The best yeast extract may vary from strain to strain. For each strain, the specific yeast extract should be selected based on the knowledge of the person skilled in the art. Yeast extract is preferably present in an amount of 2 to 6wt%, based on the total weight of the growth medium.

A carbohydrate is also needed for the lactic acid bacteria to grow. Diverse carbohydrates can be used, such as oligosaccharides, polysaccharides, sugars and mixtures thereof. Such carbohydrates are used as carbon source by the bacteria. Preferably, the at least one carbohydrate is at least on sugar, such as at least one mono-or disaccharide. Such sugar may be any sugar commonly used in growth media for lactic acid bacteria and are well-known to the person skilled in the art. Depending on the strain to be cultured, it may be useful to use a combination of two or more carbohydrates, preferably two or more sugars. Preferably the at least one sugar is selected from glucose, fructose, sucrose, lactose and combinations thereof, as these sugars are particularly effective source of nutrients for the culture of lactic acid bacteria. The total concentration of the at least one carbohydrate, preferably of the at least one sugar, is preferably of 1 to 8wt%, based on the total weight of the growth medium.

The bacterial growth medium may comprise further ingredients, which are well known to the person skilled in the art, such as peptone or salts. Any peptone commonly used in MRS-type media can be used, such as yeast peptone, meat peptone and casein peptone. Not all strains need a peptone. When it is desired to add peptone, the preferred peptone to be used varies from one strain to another. The person skilled in the art of growing a particular strain knows well which types of peptones are preferred. Peptone is in particular advantageous for strains that are sensitive to drying process, in particular to drying processes involving the use of heat, such as spray-drying, because the use of peptone in the growth medium has proven efficient to make the strains more resistant during such processes. Peptone is preferably present in an amount of 0.1 to 7wt%, based on the total weight of the growth medium. Examples of salts that can be advantageously present in the growth medium include calcium carbonate, di-ammonium hydrogen citrate, sodium acetate, MgSO₄, MnSO₄ or Na₂HPO₄. The choice of salt(s) to be used depends on the strain to be produced and is well-known to the person skilled in the art.

In an embodiment of the invention, the growth medium does not comprise milk.

### Process for preparing a bacterial growth medium

The growth medium of the present invention can be prepared by a process comprising adding lecithin, yeast extract and at least one carbohydrate to water to form an aqueous dispersion. Additional ingredients can be added, such as those described in the section related to the growth medium. The lecithin, yeast extract and carbohydrate are preferably as defined above in any embodiment of the growth medium and are preferably added in the amounts described in that section.

In a preferred aspect, the process does not comprise a step of adding polyoxyethylene-sorbitan-mono-oleate.

### Process for producing a lactic acid bacteria biomass

The process of the present invention for producing a lactic acid bacteria biomass comprises culturing the bacteria with a growth medium according to any of the above-described embodiments and harvesting the bacteria from the growth medium.

The lactic acid bacteria is preferably of the *Lactobacillus* genus. More preferably, it is selected from *Lactobacillus johnsonii, Lactobacillus acidophilus, Lactobacillus bulgaricus, Lactobacillus paracasei, Lactobacillus fermentum, Lactobacillus delbrueckii, Lactobacillus reuteri* or *Lactobacillus curvatus.* Most preferably, the lactic acid bacteria is selected from *Lactobacillus johnsonii* deposited as CNCM I-1225, *Lactobacillus curvatus* deposited as deposited as ATCC 25601, *Lactobacillus bulgaricus* deposited as ATCC 11842 or *Lactobacillus acidophilus* deposited as ATCC4356. In another embodiment, the lactic acid bacteria is a probiotic bacteria, preferably a probiotic bacteria of the *Lactobacillus* genus and more preferably a probiotic bacteria selected from the above listed species and strains. Probiotic bacteria are defined as bacterial cell preparations with a beneficial effect on the health or well-being of the host (Salminen S, Ouwehand A. Benno Y. et al., Probiotics: how should they be defined, Trends Food Sci. Technol. 1999:10 107-10).

The fermentation step is carried out in a way that is well known to the person skilled in the art. For each strain, suitable yields can be obtained with the fermentation medium of the present invention, without changing the fermentation conditions compared to what the person skilled in the art would use for fermentation of the same strain with a prior art growth medium. Thus, for a given strain, the same fermentation conditions can be used, as with a growth medium comprising Tween^{®} 80, for example with the MRS medium.

The fermentation may be carried out under anaerobic or aerobic conditions, depending on the strain to be produced. Also, the pH may be controlled or not, depending on the conditions known to be the best for a specific strain to grow. The temperature and duration of the fermentation step is variable from one strain to another and is also well-known to the person skilled in the art of lactic acid bacteria fermentation.

The harvesting step is also carried out in a way that is well known to the person skilled in the art, such as by concentrating the growth medium. Preferably, the harvesting step comprises a washing step.

Optionally the lactic acid bacteria are dried after being harvested. The drying step may be carried out using any known method, such as spray-drying, fluid bed drying, air convective drying, atmospheric drying, roller drying or freeze drying and more preferably spray-drying.

Optionally, the bacteria may further be admixed with protective agents and/or carriers before the drying step, as known to the person skilled in the art and as appropriate depending on the drying method to be used and the bacteria to be dried.

### Lactic acid bacteria biomass

A lactic acid bacteria biomass obtainable or obtained by the process of the invention is also an object of the present invention. The process is defined according to any of the above-described embodiments.

Harvested bacteria usually retain traces of the components of the growth medium, even after washing. Thus, in an embodiment, the invention relates to a lactic acid bacteria biomass comprising a concentrated lactic acid bacteria, lecithin, yeast extract and at least one carbohydrate. The lecithin, yeast extract and at least one carbohydrate are preferably present in trace amounts. Lecithin, yeast extract and carbohydrates being associated with no particular health risk in small amounts, it is not problematic that these ingredients remain as traces in the biomass.

To the contrary, traces of polyoxyethylene-sorbitan-mono-oleate that can be found in known bacterial biomass produced with a medium comprising this substance are undesirable. It is advantageous that the biomass of the invention comprises as little an amount of polyoxyethylene-sorbitan-mono-oleate as possible. Preferably the lactic acid bacteria is substantially, more preferably completely, devoid of polyoxyethylene-sorbitan-mono-oleate.

The lactic acid bacteria, lecithin, yeast extract and carbohydrate are as defined above, in any embodiment of the invention.

### Bacterial culture

The invention also encompasses a bacterial culture comprising lactic acid bacteria in a bacterial growth medium of the invention. The lactic acid bacteria and the growth medium are defined as described above in any embodiment of the invention. As the growth medium of the present invention is particularly efficient to obtain high yield of bacteria, the amount of lactic acid bacteria in the bacterial culture of the invention is typically of at least 1 E+7 cfu/mL, preferably at least 1 E+8 cfu/mL, more preferably at least 3E+8 cfu/mL, even more preferably at least 1 E+9 cfu/mL, most preferably at least 4E+9 cfu/mL.

### Use of lecithin to stimulate the growth of lactic acid bacteria

The invention also relates to the use of lecithin to stimulate the growth of lactic acid bacteria in a growth medium, wherein the growth medium comprises yeast extract and at least one carbohydrate and comprises less than 0.1wt% of polyoxyethylene-sorbitan-mono-oleate.

Preferably, the growth medium comprises less than 0.05wt%, more preferably less than 0.01wt% of polyoxyethylene-sorbitan-mono-oleate. Most preferably it is completely devoid of polyoxyethylene-sorbitan-mono-oleate.

As shown below in the examples, lecithin is effective to stimulate the growth of lactic acid bacteria in a growth medium comprising yeast extract and at least one carbohydrate. The yield of bacteria fermented in a medium comprising lecithin is significantly higher than the yield obtained when the same bacteria is fermented in the same medium but without lecithin. This applies to a large diversity of lactic acid bacteria, for example of the *Lactobacillus* genus. The yield obtained when lecithin is used is similar or higher than the yield obtained when Tween^{®} 80 is used instead of lecithin. Lecithin is preferably as described above in any embodiment of the invention and it is preferably used in an amount as described above.

Yields as high as 1 E+7 cfu/mL, preferably at least 1 E+8 cfu/mL, more preferably at least 3E+8 cfu/mL, even more preferably at least 1 E+9 cfu/mL, most preferably at least 4E+9 cfu/mL are obtained when lactic acid bacteria are grown using lecithin in the growth medium. Thus the invention preferably relates to the use of a growth medium comprising lecithin, yeast extract and at least one carbohydrate for the production of a lactic acid bacteria culture comprising at least 1E+7 cfu/mL, preferably at least 1E+8 cfu/mL, more preferably at least 3E+8 cfu/mL, even more preferably at least 1E+9 cfu/mL, most preferably at least 4E+9 cfu/mL.

### Use of lecithin to improve the survival of lactic acid bacteria

The present inventors have also found that the use of lecithin in the growth medium of lactic acid bacteria was successful in improving the survival of the obtained lactic acid bacteria during drying, storage and/or reconstitution with a liquid. Thus the invention relates to the use of a lecithin to improve the survival of lactic acid bacteria during drying, storage and/or reconstitution with a liquid, wherein the lecithin is added to the growth medium of the lactic acid bacteria, wherein the growth medium comprises yeast extract and at least one carbohydrate and wherein the growth medium comprises less than 0.1wt% of polyoxyethylene-sorbitan-mono-oleate.

Preferably, the growth medium comprises less than 0.05wt%, more preferably less than 0.01wt% of polyoxyethylene-sorbitan-mono-oleate. Most preferably it is completely devoid of polyoxyethylene-sorbitan-mono-oleate.

Preferably the growth medium is a growth medium comprising lecithin, yeast extract and at least one carbohydrate, as described above in any of the embodiments of the invention.

By fermenting the lactic acid bacteria in the growth medium, the lactic acid bacteria acquires improved resistance during drying, storage and/or reconstitution with a liquid.

The lactic acid bacteria has improved resistance to any drying step such as for example spray-drying, fluid bed drying, air convective drying, atmospheric drying, roller drying or freeze drying. The use of lecithin is particularly efficient to protect the lactic acid bacteria during a drying step involving the use of heat, such as spray-drying, fluid bed drying, air convective drying or atmospheric drying. It is most efficient to protect lactic acid bacteria during a spray-drying process.

The use of lecithin in the growth medium is also effective in protecting the lactic acid bacteria during storage, preferably storage in powder form subsequent to a drying step, preferably subsequent to a spray-drying step. This is also effective to protect dried lactic acid bacteria upon reconstitution with a liquid.

Protection of the lactic acid bacteria is intended here as the reduction of the loss of viable bacteria during drying, storage and/or reconstitution with a liquid.

In another embodiment, the invention provides a method for improving the survival of lactic acid bacteria during drying, during storage and/or upon reconstitution with a liquid comprising the steps of
a) fermenting the lactic acid bacteria in a growth medium comprising yeast extract and at least one carbohydrate; and
b) harvesting the lactic acid bacteria after fermentation,
wherein the growth medium comprises lecithin and comprises less than 0.1wt% of polyoxyethylene-sorbitan-mono-oleate.

Preferably, the growth medium comprises less than 0.05wt%, more preferably less than 0.01wt% of polyoxyethylene-sorbitan-mono-oleate. Most preferably it is completely devoid of polyoxyethylene-sorbitan-mono-oleate.

In another embodiment, the invention provides a method for improving the survival of lactic acid bacteria during a drying step comprising the steps of
a) fermenting the lactic acid bacteria in a growth medium comprising yeast extract and at least one carbohydrate;
b) harvesting the lactic acid bacteria after fermentation; and
c) drying the harvested lactic acid bacteria,
wherein the growth medium comprises lecithin and is devoid of polyoxyethylene-sorbitan-mono-oleate.

In another embodiment, the invention provides a method for improving the survival of a lactic acid bacteria during storage comprising the steps of
a) fermenting the lactic acid bacteria in a growth medium comprising yeast extract and at least one carbohydrate;
b) harvesting the lactic acid bacteria after fermentation;
c) drying the harvested lactic acid bacteria; and
d) storing the dried lactic acid bacteria, preferably at ambient temperature and pressure,
wherein the growth medium comprises lecithin and comprises less than 0.1wt% of polyoxyethylene-sorbitan-mono-oleate.

Preferably, the growth medium comprises less than 0.05wt%, more preferably less than 0.01wt% of polyoxyethylene-sorbitan-mono-oleate. Most preferably it is completely devoid of polyoxyethylene-sorbitan-mono-oleate.

In still another embodiment, the invention provides a method for improving the survival of a lactic acid bacteria during reconstitution with a liquid comprising the steps of
a) fermenting the lactic acid bacteria in a growth medium comprising yeast extract and at least one carbohydrate;
b) harvesting the lactic acid bacteria after fermentation;
c) drying the harvested lactic acid bacteria;
d) optionally storing the dried lactic acid bacteria; and
e) reconstituting the dried lactic acid bacteria in liquid,
wherein the growth medium comprises lecithin and comprises less than 0.1wt% of polyoxyethylene-sorbitan-mono-oleate.

Preferably, the growth medium comprises less than 0.05wt%, more preferably less than 0.01wt% of polyoxyethylene-sorbitan-mono-oleate. Most preferably it is completely devoid of polyoxyethylene-sorbitan-mono-oleate.

In any of the above described methods, the growth medium, the lactic acid bacteria, the lecithin and the process steps are defined according to any of the above-described embodiments of the invention.

In a preferred embodiment, the liquid, as mentioned in any of the above embodiments is an aqueous liquid, preferably water, more preferably water above room temperature.

### Product

The present invention also provides a product comprising the lactic acid bacteria biomass according to any of the above-described embodiments. The product can be any type of product in which lactic acid bacteria can be incorporated, such as a food or beverage product, an animal feed product, a nutritional supplement for human or animal, a pharmaceutical composition or a cosmetic composition. The product may be solid or liquid. Preferably, the product may be intended to be used by the final consumer in solid (such as powder form) or semi-solid form (such as for example in the form of a paste) or, alternatively, to be reconstituted into a liquid before use.

Food and beverage products include all products intended to be consumed orally by human beings, for the purpose of providing nutrition and/or pleasure. It can for example be a nutritional composition, such as for infants and/or young children, for a pregnant or lactating woman or a woman desiring to get pregnant, for individuals in need of a special nutrition due to an adverse health condition or for elderly people. More preferably, the nutritional composition is selected from infant formula, infant cereals, follow-up formula, growing-up milks, functional milks and milk products for pregnant and lactating women or for women desiring to get pregnant. Other examples of food and beverage products include sweet and savoury snacks, powdered drinks, cereal products and dairy products, such as milk products or yogurts. In an embodiment, the product is not a fermented product. More preferably, the product is an infant formula, a follow-on formula, a growing-up milk or a product for pregnant or lactating women. Most preferably it is an infant formula.

The product can also be in the form of an animal food product or a nutritional supplement for animals. Preferably, the animal is a mammal. Examples of animals include, cows, sheep, goats, horses, dogs, cats, rabbits, rats, mice, fish, birds and the like.

Nutritional supplements are typically present in the form of a liquid, such as a refrigerated liquid, of a powder or of a tablet or capsule. Preferably it is in the form of a powder, a tablet or a capsule. Powder supplements typically encompass supplements to be dissolved in a liquid or to be sprinkled on food or in a beverage. Such supplements are intended to provide additional nutrients and/or a health benefit to the subject consuming it, as well as other beneficial ingredients, such as beneficial microorganisms, for example probiotic bacteria. A supplement according to the present invention can be used for providing nutrients and/or a health benefit to human beings, as well as to animals, as defined above. Nutritional supplements include for example powder supplements to be added to breast milk, for example for premature or low birth weight infants. It also includes supplements for pregnant or lactating woman or for woman desiring to get pregnant.

Pharmaceutical products include powder, tablet or capsule products intended to treat or prevent an adverse medical condition in a subject in need thereof, or to promote a favourable health condition.

Cosmetic compositions are typically intended for an aesthetic effect on the body and may be for topical use or may be administered by oral route, in the form of a powder, tablet or capsule.

The product of the present invention preferably comprises live microorganisms, preferably beneficial bacteria, such as probiotic bacteria, in an amount of at least 5E+06 CFU per gram of product, on a dry weight basis.

The present invention will now be described in further details by the way of the following examples.

### Example 1: Production of Lactobacillus johnsonii (CNCM I-1225)

Growth Media A to G were prepared with the ingredients listed in Table 1 below.

**Table 1: Composition of growth Media A to G**

| | **Concentration (g/L)** | | | | | | |
|---|---|---|---|---|---|---|---|
| **Ingredient** | **A** | **B** | **C** | **D** | **E** | **F** | **G** |
| Yeast extract 4101 | 30.00 | 30.00 | 30.00 | 30.00 | 30.00 | 30.00 | 30.00 |
| Yeast peptone HypA | 5.00 | 5.00 | 5.00 | 5.00 | 5.00 | 5.00 | 5.00 |
| Glucose | 25.00 | 25.00 | 25.00 | 25.00 | 25.00 | 25.00 | 25.00 |
| Fructose | 25.00 | 25.00 | 25.00 | 25.00 | 25.00 | 25.00 | 25.00 |
| Tween^{®} 80 | 3.00 | 0.00 | 0.00 | 0.00 | 0.00 | 0.00 | 0.00 |
| Lecithin¹⁾ | 0.00 | 1.00 | 2.00 | 3.00 | 4.00 | 5.00 | 6.00 |
| Calcium carbonate | 7.50 | 7.50 | 7.50 | 7.50 | 7.50 | 7.50 | 7.50 |
| Distilled water | 1L | 1L | 1L | 1L | 1L | 1L | 1L |

| | | | | | | | |
|---|---|---|---|---|---|---|---|
| *1) De-oiled lecithin in powder form Emulpur^{®} IP (origin: Cargill)* | | | | | | | |

The fermentation media were prepared by admixing the ingredients listed in Table 1 and were sterilized by autoclavation at 121°C for 15 minutes. The fermentation was performed in a 1 L DasGip system (Eppendorf, Germany), in 600mL fermentation volume at 40°C. The anaerobic conditions were maintained with CO₂ in the headspace. Partial pH control was achieved by the presence of calcium carbonate in the fermentation medium. Fermentation was completed once pH drop below 4.1 was achieved. The bacteria were then harvested.

Then, the cell count analysis was carried out by classical plating method on MRS agar plates and subsequent incubation at 37°C for 48 hours in accordance with the methods described in Monroe Jay J., Loessner MJ., Golden DA., Modern Food 10 Microbiology, 7th edition, Springer Science, New York, N.Y.

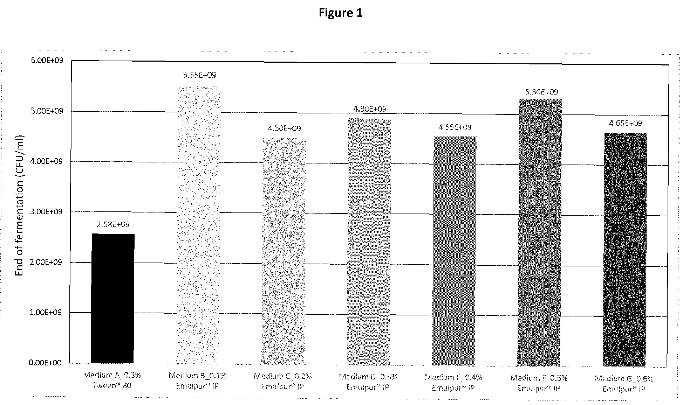

The results of the cell count analysis are provided in Figure 1. Growth Media B to G (according to the invention), which comprise lecithin as a replacement of Tween^{®} 80, were all successful in obtaining a higher fermentation yield (higher cell count) than the prior art medium A, comprising a standard amount of Tween^{®} 80. The yield was also much higher than in a medium devoid of both Tween^{®} 80 and lecithin, as the strain was unable to grow in a suitable way in a medium devoid of Tween^{®} 80 and lecithin (yield of 3x10⁶ cfu/mL only at end of fermentation, which is more than 3 log less than the yield obtained with Medium B of the invention). These results show that not only *Lactobacillus johnsonii* is able to grow in a growth medium in which Tween^{®} 80 has been replaced by lecithin, but also the growth medium of the invention surprisingly achieved better yield than the prior art medium.

### Example 2: Production of Lactobacillus bulgaricus (ATCC11842)

Growth Media H to L were prepared with the ingredients listed in Table 2 below.

**Table 2: Composition of growth Media H to L**

| | **Concentration (g/L)** | | | | |
|---|---|---|---|---|---|
| **Ingredient** | **H** | **I** | **J** | **K** | **L** |
| Bacto Proteose Peptone n°3 | 10.00 | 10.00 | 10.00 | 10.00 | 10.00 |
| Bacto Yeast Extract | 5.00 | 5.00 | 5.00 | 5.00 | 5.00 |
| Di-ammonium hydroqen citrate | 2.00 | 2.00 | 2.00 | 2.00 | 2.00 |
| Sodium acetate | 5.00 | 5.00 | 5.00 | 5.00 | 5.00 |
| MgSO₄ | 0.10 | 0.10 | 0.10 | 0.10 | 0.10 |
| MnSO₄ | 0.05 | 0.05 | 0.05 | 0.05 | 0.05 |
| Na₂HPO₄ | 2.00 | 2.00 | 2.00 | 2.00 | 2.00 |
| Glucose | 20.00 | 20.00 | 20.00 | 20.00 | 20.00 |
| Tween^{®} 80 | 0.00 | 1.00 | 0.00 | 0.00 | 0.00 |
| Lecithin¹⁾ | 0.00 | 0.00 | 1.00 | 2.00 | 3.00 |
| Distilled water | 1L | 1L | 1L | 1L | 1L |

| | | | | | |
|---|---|---|---|---|---|
| *1) De-oiled lecithin in powder form Emulpur IP (origin: Cargill)* | | | | | |

The fermentation media were prepared by admixing the ingredients listed in Table 2 and were sterilized by autoclavation at 121°C for 15 minutes. The fermentation was performed in CINAC system (Alliance Instruments, France), in 100mL fermentation volume. The pH was not controlled and fermentation was performed under aerobic conditions at 37°C. Fermentation was completed when no further pH decrease was observed.

Then, the cell count analysis was carried out by classical plating method on MRS agar plates and subsequent incubation at 37°C for 48 hours in accordance with the methods described in Monroe Jay J., Loessner MJ., Golden DA., Modern Food 10 Microbiology, 7th edition, Springer Science, New York, N.Y.

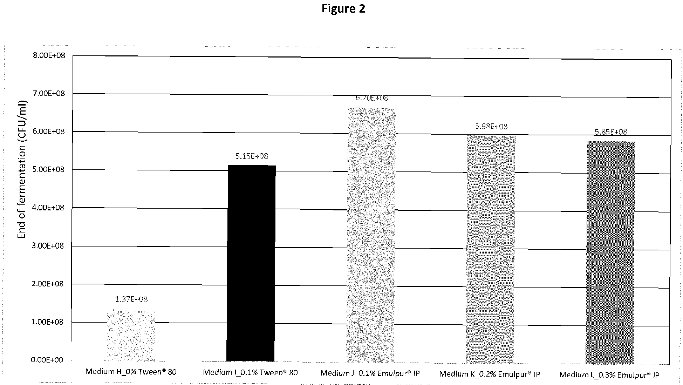

The results of the cell count analysis are provided in Figure 2. Growth Media J to L (according to the invention), which comprise lecithin as a replacement of Tween^{®} 80, were all successful in obtaining similar and even slightly higher fermentation yield (slightly higher cell count) than the prior art medium I, comprising a standard amount of Tween^{®} 80. The yield was also much higher than in medium H, which is devoid of both Tween^{®} 80 and lecithin. These results show that *Lactobacillus bulgaricus* is advantageously able to grow with good yield in a growth medium in which Tween^{®} 80 has been replaced by lecithin and even a slightly better yield is obtained than with standard amount of Tween^{®} 80.

### Example 3: Production of Lactobacillus curvatus (ATCC 25601)

Fermentation Media H to L were prepared as described in Example 2 and were sterilized by autoclavation at 121°C for 15 minutes. The fermentation was performed in CINAC system (Alliance Instruments, France), in 100mL fermentation volume. The pH was not controlled and fermentation was performed under aerobic conditions at 30°C. Fermentation was completed when no further pH decrease was observed.

Then, the cell count analysis was carried out by classical plating method on MRS agar plates and subsequent incubation at 30°C for 48 hours in accordance with the methods described in Monroe Jay J., Loessner MJ., Golden DA., Modern Food 10 Microbiology, 7th edition, Springer Science, New York, N.Y.

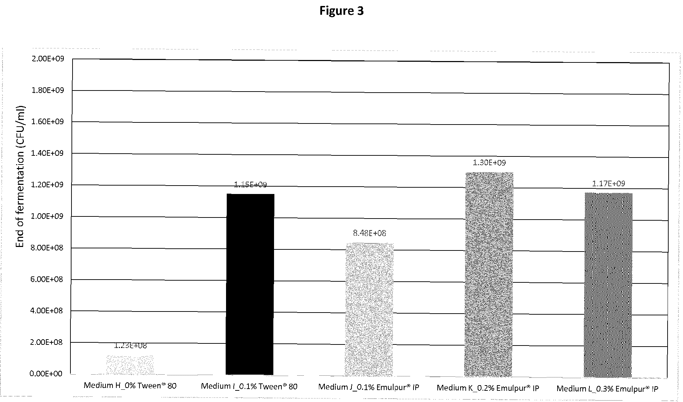

The results of the cell count analysis are provided in Figure 3. Growth Media J to L (according to the invention), which comprise lecithin as a replacement of Tween^{®} 80, were all successful in obtaining similar fermentation yield as the prior art Medium I, comprising a standard amount of Tween^{®} 80. The yield was much higher than in Medium H, which is devoid of both Tween^{®} 80 and lecithin. These results show that *Lactobacillus bulgaricus* is advantageously able to grow with good yield in a growth medium in which Tween^{®} 80 has been replaced by lecithin.

### Example 4: Production of Lactobacillus acidophilus (ATCC 4356)

Fermentation Media H to L were prepared as described in Example 2 and were sterilized by autoclavation at 121°C for 15 minutes. The fermentation was performed in CINAC system (Alliance Instruments, France), in 100mL fermentation volume. The pH was not controlled and fermentation was performed under aerobic conditions at 37°C. Fermentation was completed when no further pH decrease was observed.

Then, the cell count analysis was carried out by classical plating method on MRS agar plates and subsequent incubation at 37°C for 48 hours in accordance with the methods described in Monroe Jay J., Loessner MJ., Golden DA., Modern Food 10 Microbiology, 7th edition, Springer Science, New York, N.Y.

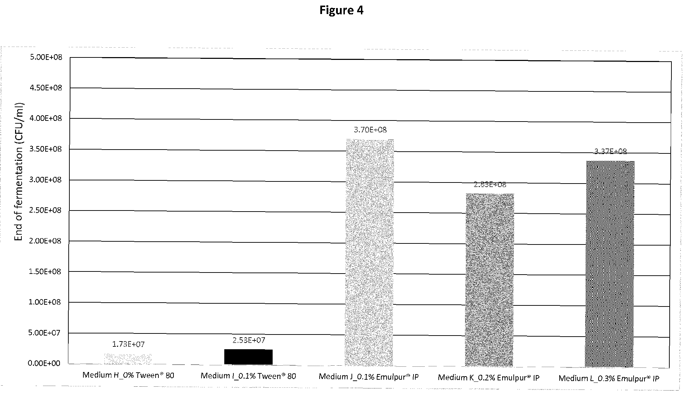

The results of the cell count analysis are provided in Figure 4. Growth Media J to L (according to the invention), which comprise lecithin as a replacement of Tween^{®} 80, were all successful in obtaining significantly higher fermentation yield (>1 log higher) than the prior art medium I, comprising a standard amount of Tween^{®} 80. The yield was also much higher than in Medium H, which is devoid of both Tween^{®} 80 and lecithin. These results show that *Lactobacillus acidophilus* is advantageously able to grow with improved yield in a growth medium in which Tween^{®} 80 has been replaced by lecithin.

### Example 5: Production of Lactobacillus johnsonii (CNCM I-1225) with different lecithins

Growth Media M to Q were prepared with the ingredients listed in Table 3 below.

**Table 3: Composition of growth Media M to Q**

| | **Concentration (g/L)** | |
|---|---|---|
| **Ingredient** | **M to P** | **Q** |
| Yeast extract 4101 | 30.00 | 30.00 |
| Yeast peptone HypA | 5.00 | 5.00 |
| Glucose | 25.00 | 25.00 |
| Fructose | 25.00 | 25.00 |
| Tween^{®} 80 | 0.00 | 1.00 |
| Lecithin¹⁾ | 1.00 | 0.00 |
| Calcium carbonate | 7.50 | 7.50 |
| Distilled water | 1L | 1L |

| | | |
|---|---|---|
| *1) Emultop^{®} IP was used for Medium M, Emulpur^{®} SF was used for Medium N, Emulfluid^{®} NGM was used for Medium O, Emulfluid^{®} F 30 IP was used for Medium P. All lecithins were sourced from Cargill.* | | |

The features of the different lecithin used in the present trials are summarized in Table 4 below.

**Table 4: Characteristics of the lecithin sources used**

| **Lecithin** | **Origin** | **De-oiled** | **Format** | **Hydrolysis** | **Composition** |
|---|---|---|---|---|---|
| Emulpur^{®} IP | Soy | Yes | Powder | No | Lecithin with purity >95%: Mixture of complex lipids and small amount of carbohydrate |
| Emulpur^{®} SF | Sunflower | Yes | Powder | No | Lecithin with purity >95%: Mixture of complex lipids and small amount of carbohydrate |
| Emultop^{®} IP | Soy | Yes | Powder | Yes | Lecithin with purity >95%: Mixture of complex lipids and small amount of carbohydrate |
| Emulfluid^{®} NGM | Soy | No | Liquid | Yes | Lecithin with purity >50%: Mixture of complex lipids and small amount of carbohydrate |
| Emulfluid^{®} F30 *IP* | Soy | No | Liquid | No | Lecithin with purity >95%: Mixture of complex lipids and small amount of carbohydrate; enriched in phosphatidylcholine. |

The fermentation media B and M to Q were prepared by admixing the ingredients listed in Table 2 and 3, respectively, and were sterilized by autoclavation at 121°C for 15 minutes. The fermentation was performed in a 1 L DasGip system (Eppendorf, Germany), in 600mL fermentation volume at 40°C. The anaerobic conditions were maintained with CO₂ in the headspace. Partial pH control was achieved by the presence of calcium carbonate in the fermentation medium. Fermentation was completed once pH drop below 4.1 was achieved. The bacteria were then harvested. This method was carried out with each of the Media B and M to Q, in order to compare the yields of a standard medium with Tween^{®} 80 (prior art, Medium Q), with the yield obtained with five different lecithin types (according to the invention, Media B and M to P).

The cell count analysis was carried out by classical plating method on MRS agar plates and subsequent incubation at 37°C for 48 hours in accordance with the methods described in Monroe Jay J., Loessner MJ., Golden DA., Modern Food 10 Microbiology, 7th edition, Springer Science, New York, N.Y.

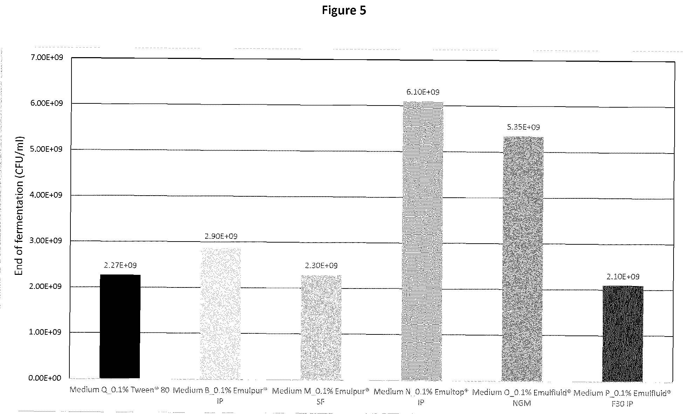

The results of the cell count analysis are provided in Figure 5. Growth Media B and M to P (according to the invention), which comprise lecithin as a replacement of Tween^{®} 80, were all successful in obtaining similar or higher fermentation yield (similar or higher cell count) than the prior art Medium Q, comprising a standard amount of Tween^{®} 80. The yield was also much higher than in a medium devoid of both Tween^{®} 80 and lecithin, as the strain was unable to grow in a suitable way in a growth medium devoid of both Tween^{®} 80 and lecithin (yield of 3x10⁶ cfu/mL only, at end of fermentation, which is about 3 log less than the yield obtained with Media B and M to P according to the invention). These results show that a wide range of different types of lecithin can be used to successfully replace Tween^{®} 80 in a growth medium for *Lactobacillus johnsonii.*

## Claims

1. A bacterial growth medium comprising yeast extract, at least one carbohydrate and lecithin.

2. A bacterial growth medium according to claim 1, which comprises less than 0.1wt% of polyoxyethylene-sorbitan-mono-oleate.

3. A bacterial growth medium according to claim 2, which is devoid of polyoxyethylene-sorbitan-mono-oleate.

4. A bacterial growth medium according to any one of the previous claims, wherein the lecithin is de-oiled lecithin in powder form.

5. A bacterial growth medium according to any one of the previous claims, wherein the lecithin is present in an amount of at least 0.1wt%, based on the total weight of the growth medium.

6. A process for producing a lactic acid bacteria biomass comprising
a. fermenting at least one strain of lactic acid bacteria in a bacterial growth medium according to any one of the preceding claims; and
b. harvesting the cultured bacteria.

7. A process according to claim 6, wherein the lactic acid bacteria is of the *Lactobacillus* genus.

8. A process according to claim 7, wherein the lactic acid bacteria is selected from *Lactobacillus johnsonii, Lactobacillus acidophilus, Lactobacillus bulgaricus, Lactobacillus fermentum, Lactobacillus delbrueckii, Lactobacillus reuteri, lactobacillus paracasei* or *Lactobacillus curvatus.*

9. A process according to claim 8, wherein the lactic acid bacteria is selected from *Lactobacillus johnsonii* deposited as CNCM I-1225, *Lactobacillus curvatus* deposited as deposited as ATCC 25601, *Lactobacillus bulgaricus* deposited as ATCC 11842 or *Lactobacillus acidophilus* deposited as ATCC 4356.

10. A bacterial culture comprising lactic acid bacteria in a bacterial growth medium according to any one of claims 1 to 5.

11. Use of lecithin to stimulate the growth of lactic acid bacteria in a growth medium, wherein the growth medium comprises yeast extract and at least one carbohydrate and comprises less than 0.1wt% of polyoxyethylene-sorbitan-mono-oleate.

12. A lactic acid bacteria biomass comprising a concentrated lactic acid bacteria, lecithin, yeast extract and at least one carbohydrate.

13. A product comprising a lactic acid bacteria biomass according to claim 12.

14. A product according to claim 13, which is a nutritional composition selected from infant formula, infant cereals, follow-up formula, growing-up milks, functional milks and milk products for pregnant and lactating women.

15. Use of a lecithin to improve the survival of lactic acid bacteria during drying, storage and/or reconstitution with a liquid, wherein the lecithin is added to the growth medium of the lactic acid bacteria, wherein the growth medium comprises yeast extract and at least one carbohydrate and wherein the growth medium comprises less than 0.1wt% of polyoxyethylene-sorbitan-mono-oleate.
